Europäisches Patentamt

(19) European Patent Office

Office européen des brevets

(11) Veröffentlichungsnummer: **0 105 429 B1**

(12) **EUROPÄISCHE PATENTSCHRIFT**

(45) Veröffentlichungstag der Patentschrift: **18.02.87**

(21) Anmeldenummer: **83109522.9**

(22) Anmeldetag: **24.09.83**

(51) Int. Cl.⁴: **A 61 F 13/20**

(54) **Tampon für die Frauenhygiene und Verfahren zu dessen Herstellung.**

(30) Priorität: **02.10.82 DE 3236541**

(43) Veröffentlichungstag der Anmeldung:
**18.04.84 Patentblatt 84/16**

(45) Bekanntmachung des Hinweises auf die
Patenterteilung:
**18.02.87 Patentblatt 87/08**

(84) Benannte Vertragsstaaten:
**AT BE CH DE FR GB IT LI NL SE**

(56) Entgegenhaltungen:
**DE-A-2 855 179**
**DE-B-2 445 214**

(73) Patentinhaber: **Vereinigte Papierwerke AG**
**Schoppershofstrasse 80**
**D-8500 Nürnberg (DE)**

(72) Erfinder: **Sustmann, Scarlet, Dr.**
**Am Nachtigallenwäldchen 34**
**D-4060 Viersen 12 (DE)**

(74) Vertreter: **Pohl, Hans Ludwig**
**Hefnersplatz 3**
**D-8500 Nürnberg 11 (DE)**

Courier Press, Leamington Spa, England.

EP 0 105 429 B1

**Beschreibung**

Die Erfindung betrifft ein Verfahren zum kontinuierlichen Herstellen von Tampons für die Frauenhygiene, die aus einem in flüssigkeitsdurchlässigem Vliesmaterial eingeschlossenen Wattebandabschnitt bestehen, wobei das Vliesmaterial zumindest zum Teil aus thermoplastischen Fasern besteht und siegelfähig ist und nach dem Zusammenbringen mit dem Wattebandabschnitt versiegelt und gestanzt wird und die von dem Vliesmaterial eingeschlossenen Wattebandabschnitte mit je einem Rückholfaden ausgestattet und zu zylinderfömigen Körpern radial und/oder axial in bezug auf den Rückholfaden verpreßt werden.

Aus DE—A—28 55 179 ist ein gattungsgemäßes Verfahren zum Herstellen von Tampons mit Rückholband bekannt, bei welchem Saugstoff von einer Hülle aus flüssigkeitsdurchlassigem, verschweißbarem Vliesmaterial umschlossen und in eine etwa zylindrische Form gepreßt wird. Dabei bildet die Hülle mit dem Saugstoff ein flaches Kissen, dessen vorderer und hinterer Rand auf die Oberseite des Kissens gefaltet werden und mit ihren Enden sich gegenüberliegen. An dem Kissen ist weiterhin in dessen Längsrichtung das Rückholband angebracht, um welches als Faltlinie die beiden sich etwa senkrecht zum Rückholband erstreckenden beutelförmigen Seitenteile des Kissens gegeneinandergefaltet und in die Form des Tampons gepreßt sind.

Nachteilig bei diesem bekannten Verfahren ist die Tatsache, daß das aus einer Bahn zugeleitete Hüllmaterial durch Umlenkbleche gefaltet wird, dergestalt, daß der Saugstoff von dieser Bahn vollständig umschlossen wird. Durch diese Manipulation kann nicht ausgeschlossen werden, daß in dem Hüllmaterial in Folge Überdehnung Löcher entstehen. Durch diese Löcher wiederum kann Fasermaterial vom Saugstoff nach außen dringen und gegebenenfalls bei Gebrauch des Tampons in der Vagina zurückbleiben.

Die Aufgabe bestand deshalb darin, ein Verfahren zur Herstellung von Tampons zu schaffen, bei denen der Einschluß des Saugstoffs in flusenfreies Vliesmaterial dergestalt verbessert wird, daß Fasermaterial des Saugstoffs nicht abgelöst werden kann.

Die Lösung besteht in der Kombination der im Anspruch 1 genannten Maßnahmen.

Das als Hüllmaterial verwendete flusenfreie Vliesmaterial besteht also zumindest zum Teil aus thermoplastischen Fasern und ist an den nach dem Zusammenbringen mit dem Wattebandabschnitt offenen Kanten, den Wattebandabschnitt flusendicht einschließend, versiegelt. Bei den offenen Kanten handelt es sich beispielsweise um Schnitt- oder Stanzkanten. Das Hüllmaterial muß thermoplastische Fasernenthalten, die eine solche stoffschlüssige Verbindung, insbesondere allein durch Wärmeanwendung ermöglichen.

Es wird als erfindungsgemäß nach Art eines Sandwich-Verfahrens gearbeitet. Dabei werden die an einer Schneidstation abgetrennten Abschnitte eines Wattebandes mit Abstand voneinander auf ein Band aus dem flusenfreien Vliesmaterial gebracht. Daraufhin wird ein zweites Band aus dem Vliesmaterial auf den Wattebandabschnitt aufgebracht. Anschließend wird im so entstandenen Schichtgebilde das allseits über den Wattebandabschnitt überstehende Vliesmaterial im Raum zwischen aufeinanderfolgenden Wattebandabschnitten quer getrennt, wobei die Vliesmaterialschichten vorher oder nachher flusendicht miteinander versiegelt werden. Hierbei wird eine Walze mit mehreren nebeneinanderliegenden Siegel- und Stanzmustern verwendet. Das Trennen und umlaufende Versiegeln kann auch zugleich ausgeführt werden.

Eine zweckmäßige Ausgestaltung des erfindungsgemäßen Verfahrens besteht darin, daß ein Punktsiegelverfahren angewendet wird.

Anhand der schematischen Darstellung von Ausführungsbeispielen werden Einzelheiten der Erfindung erläutert. Es zeigen:

Fig. 1 das Einfügen einzelner Wattebandabschnitte zwischen zwei Schichten aus Vliesmaterial bei dem Sandwich-Verfahren;

Fig. 2 eine Walze zum Herstellen mehrerer umhüllter Wattebandabschnitte nebeneinander; und

Fig. 3 die Draufsicht auf eine Bahn mit mehreren, in Längs- und Querrichtung unterteilen, in flusendichtes Vliesmaterial eingesiegelten Wattebandabschnitten.

Die Vollumhüllung von Tampons wird durch den Einsatz von siegelfähigem flusenfreiem Vliesmaterial erzielt. Bevorzugt werden hierzu weiche, flexible Stoffe mit niedrigem Flächengewicht von höchstens 20 g/m², die zumindest teilweise aus thermoplastischen Fasern bestehen, verwendet.

Bei dem Verfahren werden die mit Abstand a auf ein von unten über eine Walze 21 auf ein Vliesstoffband 22 geförderten Wattebandabschnitte 23 mit einem zweiten über Walzen 24 von oben zugeführten Vliesstoffband 25 abgedeckt. Die Vliesstoffbänder 22, 25 sollen wenigstens um die doppelte Siegelnaht breiter als die Wattebandabschnitte 23 sein. Im weiteren Verlauf des Verfahrens erfolgt in einer Stanz-Siegel-Station 26 eine Rundumstanzung und -versiegelung (wobei das Siegeln vor dem Stanzen liegen kann), so daß einzelne rundum flusendicht eingehüllte Wattebandabschnitte 27 anfallen. Die weitere Verarbeitung, einschließlich des Aufbringens eines Rückholfadens, kann in üblicher Weise ausgeführt werden.

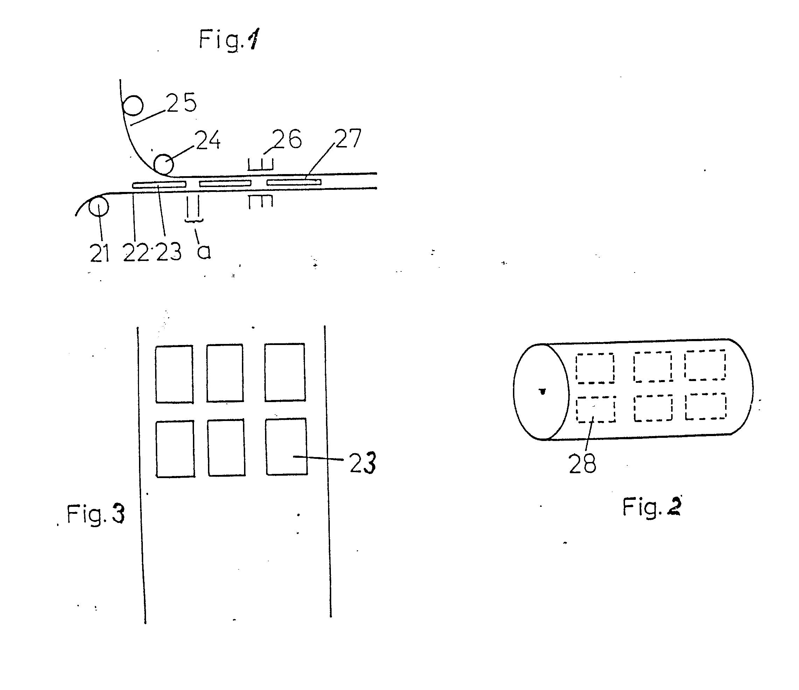

Bei dem Verfahren (Fig. 1) wird zum Stanzen und Siegeln eine Walze gemäß Fig. 2 verwendet, die auf der Oberfläche nebeneinander mehrere Muster 28 zum Siegeln und Stanzen besitzt und daher zum gleichzeitigen Herstellen mehrerer nebeneinanderliegender eingehüllter Wattebandabschnitte 23 gemäß Fig. 3 geeignet ist.

Bezugszeichenliste:
21 = Walze
22 = Vliesstoffbahn
23 = Wattebandabschnitt
24 = Walze
25 = Vliesstoffbahn
26 = Stanz/Siegel-Station
27 = eingehüllter Wattebandabschnitt
28 = Siegel-Stanzmuster

**Patentansprüche**

1. Verfahren zum kontinuierlichen Herstellen von Tampons für die Frauenhygiene, die aus einem in flüssigkeitsdurchlässigem Vliesmaterial (22, 25) eingeschlossenen Wattebandabschnitt (23) bestehen, wobei das Vliesmaterial (22, 25) zumindest zum Teil aus thermoplastischen Fasern besteht und siegelfähig ist und nach dem Zusammenbringen mit dem Wattebandabschnitt (23) versiegelt und gestanzt wird und die von dem Vliesmaterial (22, 25) eingeschlossenen Wattebandabschnitte (23) mit je einem Rückholfaden ausgestattet und zu zylinderförmigen Körpern radial und/oder axial in bezug auf den Rückholfaden verpreßt werden, dadurch gekennzeichnet, daß man zwei Lagen (22, 25) von flusenfreiem Vliesmaterial im Sandwich-Verfahren übereinander führt und auf eine Lage (22) die an einer Schneidstation abgetrennten Abschnitte (23) eines Wattebandes mit Abstand (a) voneinander aufbringt und dann die andere Lage (25) auf die Wattebandabschnitte (23) aufbringt und daß man dann die so entstandene Schichtenfolge um jeden Wattebandabschnitt (23) herum, unter Verwendung einer Walze mit mehreren nebeneinanderliegenden Siegel- und Stanzmustern (28), siegelt und zugleich, vorher oder nachher soweit erforderlich stanzt.

2. Verfahren nach Anspruch 1, dadurch gekennzeichnet, daß ein Punkt-Siegel-Verfahren angewendet wird.

**Revendications**

1. Procédé pour la fabrication continue de tampons pour l'hygiéne féminine, chaque tampon étant constitué d'un tronçon de bande d'ouate (13) enfermé dans de la matière laineuse perméable aux liquides (22, 25), cette matiére (22, 25) étant formée, au moins en partie, de fibres thermoplastiques et étant soudable, procédé dans lequel on soude et on découpe cette matière laineuse après sa réunion avec le tronçon de bande d'ouate (23) et on équipe chaque tronçon d'un fil de rappel alors qu'il est enfermé dans la matière laineuse (22, 25), et ensuite on comprime ce tronçon radialement et/ou axialement par rapport au fil de rappel pour former un corps cylindrique, ce procédé étant caractérisé par le fait que l'on guide, l'une par dessus l'autre, en un procédé sandwich, deux couches (22, 25) de matière laineuse exempte de peluches et que, sur une couche (22), on applique avec un espacement (a) entre eux, les tronçons (23) d'une bande d'ouate séparés en un poste de séparation, puis on applique l'autre couche (25) sur ces tronçons de bande d'ouate (23), et ensuite, on soude la succession de couches ainsi formée autour de chaque tronçon de bande d'ouate (23) en utilisant un rouleau pourvu de plusieurs configurations de soudage et de découpage placés les uns à côté des autres, et en même temps, auparavant ou ensuite, on découpe cette succession de couches dans la mesure où cela est nécessaire.

2. Procédé selon la revendication 1, caractérisé par le fait que l'on applique un procédé de soudage par points.

**Claims**

1. Method for the continuous manufacture of tampons for the feminine hygiene, which consist of a cotton wool tape portion (23) enclosed in a fleece material (22, 25) permeable by liquid, wherein the fleece material (22, 25) consists at least in part of thermoplastic fibres and is capable of being sealed and is sealed and punched after being brought together with the cotton wool tape portion (23) and the cotton wool tape portions (23) enclosed by the fleece material (22, 25) are each equipped with a respective retraction thread and compressed into cylindrical bodies radially and/or axially with reference to the retraction thread, characterised thereby, that one guides two layers (22, 25) of fluff-free fleece material each above the other in sandwich method and applies the portions (23) of a cotton wool tape, which were severed at a cutting station, at a spacing (a) one from the other onto a layer (22) and then applies the other layer (25) onto the cotton wool tape portions (23) and that one then, with the use of a roller with several sealing and punching patterns (28) lying one beside the other, seals and — simultaneously, previously or subsequently and in so far as required — punches the thus arisen layer sequence around each cotton wool tape portion (23).

2. Method according to claim 1, characterised thereby, that a point-sealing method is used.

Fig.*1*

21 22·23 a · · 24 · 26 · 27 · 25

Fig.3

23

Fig.*2*

28